# EUROPÄISCHE PATENTSCHRIFT

(11) **EP 0 302 850 B1**
(45) Veröffentlichungstag und Bekanntmachung des Hinweises auf die Patenterteilung: **13.01.1993**
(21) Anmeldenummer: 88890181.6
(22) Anmeldetag: 07.07.1988
(51) Int. Cl.: A61F 2/32

(54) **Künstliches Hüftgelenk**
Artificial hip joint
Articulation de hanche artificielle

(30) Priorität: 09.07.1987 AT 1738/87
(43) Veröffentlichungstag der Anmeldung: 08.02.1989
(73) Patentinhaber: Menschik, Alfred, Dr.med., A-3400 Klosterneuburg Niederösterreich (AT)
(72) Erfinder: Menschik, Alfred, Dr.med., A-3400 Klosterneuburg Niederösterreich (AT)
(74) Vertreter: Casati, Wilhelm, Dipl.-Ing.

(56) Entgegenhaltungen:
- EP-A- 0 128 036
- EP-A- 0 222 236
- EP-A- 0 226 762
- CH-A- 507 704
- DE-A- 3 247 726
- DE-B- 2 527 865
- US-A- 4 546 501

## Beschreibung

Die Erfindung betrifft ein künstliches Hüftgelenk zum Ersatz des natürlichen Gelenkskopfes und/oder der natürlichen Gelenkspfanne, wobei die künstlichen Gelenksflächen als Rotationsflächen ausgebildet sind.

Es ist bekannt, künstliche Hüftgelenke mit im wesentlichen als Kugelflächen ausgebildeten Gelenksflächen sowohl in der Gelenkspfanne als auch im Gelenkskopf herzustellen. Obwohl derartige Hüftgelenke einen guten Ersatz für krankhaft veränderte bzw. von Geburt an schlechte Hüftgelenke geboten haben, kam es immer wieder nach Implantationen künstlicher Hüftgelenke zu Gehschwierigkeiten und Behinderungen der Patienten.

Durch die CH-PS 507 704 wurde eine Schenkelkopfprothese mit einem Stiel zum Einführen in die Medullarhöhle des Femurschaftes bekannt, wobei der Stiel in Nähe seines einen Endes eine Verbreiterung mit einer Auflagefläche zum Anliegen auf dem Femoralhalsrest und an diese anschließend einen Zapfen zur Aufnahme einer Gelenkkugel aufweist. Um bei solchen Prothesen eine bessere Anpassung an die unterschiedlichen Skelettverhältnisse des Patienten zu erreichen, ohne daß durch eine Drehverbindung Veränderungen auftreten, wird in dem genannten Dokument vorgeschlagen, daß die Gelenkkugel gegenüber dem Knochen nach der endgültigen Fixierung des Stieles in der Medullarhöhle mit einem Halsteil, welcher einerseits an die Gelenkkugel und anderseits an den Auflagebund anschließt, in eine bestimmte und fixierbare Lage bringbar ist. Auch solche Prothesen können nicht voll befriedigen.

Der Erfindung liegt die Aufgabe zugrunde, hier Abhilfe oder zumindest eine Besserung des herrschenden Zustandes auf dem Gebiet der Künstlichen Hüftgelenke zu schaffen. Erreicht wird dies, wenn gemäß der Erfindung die Gelenksfläche der Gelenkspfanne bzw. die Gelenksfläche des Gelenkskopfes im Meridianschnitt als Pascal'sche Kurve (Schnecke) ausgebildet ist. Durch diese Ausgestaltung wird erreicht, daß bei jeder Abweichung zwischen den Drehachsen von Gelenkspfanne und Gelenkskopf ein Rückstellmoment auftritt, so daß jede indifferente Gleichgewichtslage, wenn man von einer einzigen Stellung absieht, in der die Achsen von Gelenkspfanne und Gelenkzkopf fluchten, vermieden ist. Der mit einem künstlichen Hüftgelenk versorgte Patient kann sohin das Gelenk der neuen Ausgestaltung besser bewegen als wenn - wie bisher - jede Lage praktisch eine indifferente Gleichgewichtslage ist.

Bevorzugt ist es, den Meridianschnitt der Gelenksfläche der Gelenkspfanne als gestreckte Pascal'sche Kurve auszubilden. Der Meridianschnitt der Gelenksfläche des Kopfes ist dann bevorzugt eine Pascal'sche Kurve mit Schlinge.

Eine weitere Ausgestaltung des erfindungsgemäßen Hüftgelenkes zeichnet sich dadurch aus, daß der Meridianschnitt der Gelenksfläche der Gelenkspfanne und der Meridianschnitt der Gelenksfläche des Gelenkskopfes Pascalkurven mit gegeneinander vertauschten Parametern sind, d.h. folgt der Meridianschnitt des Hüftkopfes der Beziehung r = a + b cos.4), so folgt der Meridianschnitt der Hüftpfanne der Beziehung r' = b + a cos. 4), wobei r bzw. r' und 4) die Polarkoordinaten eines Punktes des Meridianschnittes des Hüftkopfes bzw. der Hüftpfanne sind und 4) den Winkel zwischen der Symmetrieachse der Pascal'schen Kurve und den Radiusvektor in einem Punkt der Kurve bedeutet und a und b die vorgegebenen Parameter sind.

Das Verhältnis der beiden Parameter a/b wird bevorzugt < 1 ausgeführt. Charakteristische Werte für die Parameter sind für den Parameter a 18,75 mm und für den Parameter b 21,5 mm.

In weiterer Ausgestaltung der Erfindung kann vorgesehen werden, daß zwischen der Achse des Schenkelhalses und der Drehachse des Gelenkskopfes (gesehen in Draufsicht) eine winkelmäßige Abweichung vorhanden ist, die bevorzugt 10,7° +/- 3° beträgt. Durch diese Ausgestaltung wird erreicht, daß Totlagen zwischen Becken und Oberschenkel vermieden sind und auf den Oberschenkel immer ein Rückstellmoment wirkt. Durch die erfindungsgemäße Ausgestaltung des künstlichen Hüftgelenkes wird erreicht, daß die Drehachse des Gelenkskopfes durch die Oberschenkelknochenschaftachse verläuft, die Schenkelhalsachse jedoch bauchseitig (ventral) an der Oberschenkelschaftachse vorbeiläuft. Wiewohl diese erfindungsgemäße Gestaltung besonders zweckmäßig bei künstlichen Hüftgelenken angewendet wird, bei denen die Gelenksfläche der Gelenkspfanne bzw. die Gelenksfläche des Gelenkskopfes im Meridianschnitt als Pascal'sche Kurve (Schnecke), insbes. solchen mit den vorerwähnten Merkmalen ausgebildet ist, kann sie auch mit dem Vorteil der Vermeidung von Totlagen zwischen Becken und Oberschenkel bei künstlichen Hüftgelenken angewendet werden, bei welchen Gelenkskopf und/oder Gelenkspfanne in herkömmlicher Weise (kugelförmig) gestaltet sind.

Die Erfindung wird nachstehend anhand der Zeichnung beispielsweise näher erläutert. Es zeigen,
Fig. 1 einen Achsschnitt durch ein aus Gelenkskopf und Gelenkspfanne bestehendes, erfindungsgemäß ausgebildetes Hüftgelenk,
Fig. 2 ein Schaubild zur Erläuterung des mathematischen Zusammenhanges, und
Fig. 3 eine Draufsicht korrespondierend zu Fig. 1 mit geschnittener Gelenkspfanne.

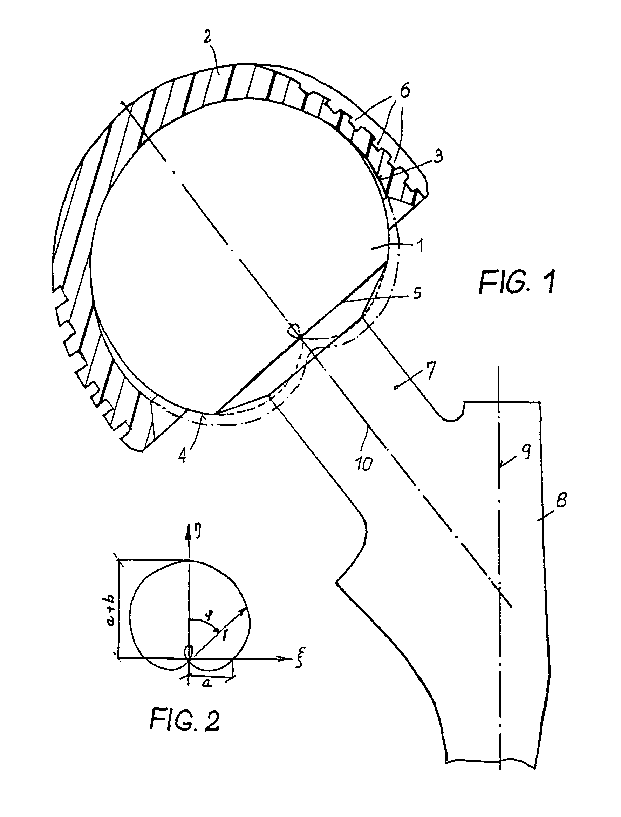

In der Zeichnung ist mit 1 der Gelenkskopf und mit 2 die Pfanne bezeichnet. Gelenkskopf 1 und Gelenkspfanne 2 sind als Drehkörper bzw. Drehflächen ausgebildet, wobei sowohl die Gelenksfläche 3 der Gelenkspfanne 2 als auch die Gelenksfläche 4 des Gelenkskopfes 1 einen von einer Pascalkurve (Pascalschnecke) gebildeten Meridianschnitt besitzen. Der Meridianschnitt der Gelenksfläche 4 des Gelenkskopfes 1 ist dabei als Pascal'sche Kurve mit Schlinge ausgebildet, wie aus Fig. 1 zu ersehen ist. Selbstverständlich wird dabei nur jener Teil der Pascal'schen Kurve verwendet, der oberhalb des Kehlkreises 5 angeordnet ist, d.i. ein Kreis, der senkrecht zur Drehachse liegt und durch den Doppelpunkt der Pascal'schen Kurve verläuft. Der Meridianschnitt der Gelenksfläche 3 der Gelenkspfanne 2 ist eine gestreckte Pascal'sche Kurve, d.h. eine Kurve, die eine auf der Symmetrieachse liegende Eindellung besitzt. Der Meridianschnitt der Gelenksfläche 3 der Gelenkspfanne 2 und der Meridianschnitt der Gelenksfläche 4 des Gelenkskopfes 1 sind Pascalkurven mit gegeneinander vertauschten Parametern.

In Fig. 2 ist zur näheren Veranschaulichung eine Pascalschnecke mit Schlinge dargestellt. In Polarkoordinaten folgen die Punkte dieser Pascal'schen Kurve der Gleichung r = a + b cos φ.

Legt man durch die Pascal'sche Kurve ein ζ/η Koordinatensystem derart, daß die -Achse mit der Symmetrieachse der Pascal'schen Kurve zusammenfällt und der Ursprung des Koordinatensystems mit dem Doppelpunkt der Pascal'schen Kurve zusammenfällt, so sind die Parameter a bzw. a + b als Achsenabschnitte der Pascal'schen Kurve festgelegt, wobei der Winkel φ als Winkel des jeweiligen Radiusvektors r gegen die Symmetrieachse (q-Achse) gemessen ist.

Folgt nun der Meridianschnitt der Hüftpfanne 2 der Beziehung r' = _{b} + a cos.φ, so folgt der Meridianschnitt des Hüftkopfes der Beziehung r = a + b cos.4). Es ergibt sich dabei, daß sowohl der Meridianschnitt der HÜftpfanne 2 als auch der Meridianschnitt des Hüftkopfes 1 auf der Symmetrieachse die gleiche Abschnittgröße, nämlich a + b, besitzen. Auf der dazu senkrechten Achse besitzt der Meridianschnitt des Hüftkopfes 1 den Achsenabschnitt a, der Meridianschnitt der Hüftpfanne 2 jedoch den Achsenabschnitt b. Die Wahl ist so getroffen, daß b> a. Bevorzugte Werte sind hiebei für a 18,75 mm und für b 21,5 mm.

Im dargestellten Ausführungsbeispiel besteht die Gelenkspfanne 2 aus Kunststoff. Am Außenmantel sind dabei in Umfangsrichtung senkrecht zur Drehachse mehrere Nuten 6 zwecks Verankerung der Gelenkspfanne im Becken mittels eines entsprechenden Zementes (Kunststoffkleber) angeordnet. Der Gelenkskopf 1 ist im dargestellten Ausführungsbeispiel als ein Stück mit Hals 7 und dem Schaft 8, der in den Oberschenkelknochen (Femur) implantiert wird, dargestellt. Es ist jedoch auch möglich, den Gelenkskopf 1 auf einen Zapfen, dessen Achse mit der Drehachse des Gelenkskopfes 1 zusammenfällt und aus einem Stück mit dem Schaft 7 besteht, aufzustecken. In einem solchen Fall kann dann der Gelenkskopf 1 auch aus Kunststoff bestehen.

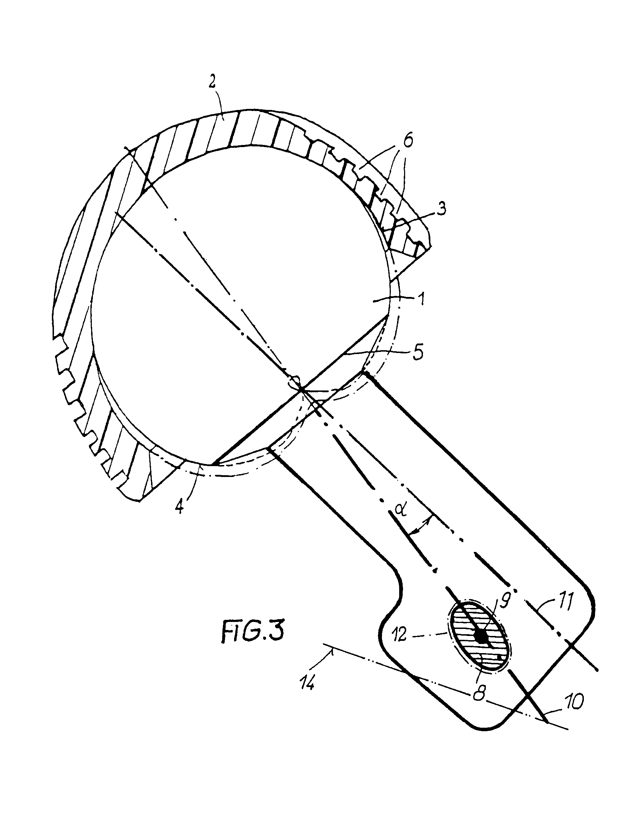

Wie aus Fig. 3 ersichtlich, ist zwischen der Achse 11 des Schwenkhalses und der Drehachse 10 des Gelenkskopfes 1, die mit der Achse 9 des Schaftes 8 des Oberschenkels bzw. der Prothese in einer Ebene liegt, eine winkelmäßige Abweichung vorhanden. Diese Abweichung beträgt bevorzugt 10,7" +/- 3°. Der Markraum 12 des Oberschenkelknochens, in welchen der Schaft 8 eingesetzt wird, ist strichpunktiert in Fig. 3 dargestellt. Die Drehachse 10 des Gelenkskopfes schließt mit der Achse 9 des Schaftes 8 einen Winkel von etwa 126 ein. In Draufsicht (Fig. 3) schließt die Drehachse 10 des Gelenkskopfes 1 mit der hinteren Femurcondylenachse 14 einen Winkel von etwa 34 ein. Die aus den Zeichnungen ersichtlichen Winkellagen der Achsen 10 und 11 (Fig. 3) bzw. 9 und 10 (Fig. 1)können auch bei künstlichen Hüftgelenken angewendet werden, deren Gelenkskopf bzw. Gelenkspfanne in herkömmlicher Weise (z.B. kugelförmig) ausgebildet sind.

## Patentansprüche

1. Künstliches Hüftgelenk zum Ersatz des natürlichen Gelenkskopfes und/oder der natürlichen Gelenkspfanne, wobei die künstlichen Gelenksflächen als Rotationsflächen ausgebildet sind, dadurch gekennzeichnet, daß die Gelenksfläche (3) der Gelenkspfanne (2) bzw. die Gelenksfläche (4) des Gelenkskopfes (1) im Meridianschnitt als Pascal'sche Kurve (Schnecke) ausgebildet ist.

2. Künstliches Hüftgelenk nach Anspruch 1, dadurch gekennzeichnet, daß der Meridianschnitt der Gelenksfläche (4) des Gelenkskopfes (1) eine Pascal'sche Kurve mit Schlinge ist.

3. Künstliches Hüftgelenk nach Anspruch 1 oder 2, dadurch gekennzeichnet, daß der Meridianschnitt der Gelenksfläche (3) der Gelenkspfanne (2) eine gestreckte Pascal'sche Kurve ist.

4. Künstliches Hüftgelenk nach einem der Ansprüche 1 bis 3, dadurch gekennzeichnet, daß der Meridianschnitt der Gelenksfläche (3) der Gelenkspfanne (2) und der Meridianschnitt der Gelenktfläche (4) des Gelenkskopfes (1) Pascalkurven mit gegeneinander vertauschten Parametern sind, d.h. folgt der Meridianschnitt des Hüftkopfes (1) der Beziehung r = a + b cos. φ, so folgt der Meridianschnitt der Hüftpfanne (2) der Beziehung r' = b + a cos. φ, wobei r bzw. r' und 4) die Polarkoordinaten eines Punktes des Meridianschnittes des Hüftkopfes bzw. der Hüftpfanne sind und 4) der Winkel zwischen der Symmetrieachse der Pascal'schen Kurve und dem Radiusvektor zu einem Punkt der Kurve bedeutet und a und b die vorgegebenen Parameter sind.

5. Künstliches Hüftgelenk nach Anspruch 4, dadurch gekennzeichnet, daß das Verhältnis a/b < 1 ist, wobei bevorzugt der Parameter a = 18,75 mm und der Parameter b = 21,5 mm ist.

6. Künstliches Hüftgelenk nach einem der Ansprüche 1 bis 5, dadurch gekennzeichnet, daß zwischen der Achse des Schenkelhalses (11) und der Drehachse (10) des Gelenkskopfes (1) (gesehen in Draufsicht) eine winkelmäßige Abweichung (a) vorhanden ist, die bevorzugt 10,7" +/- 3 beträgt.

## Claims

1. An artificial hip joint to replace the natural joint head and/or natural acetabular cavity, the artificial joint surfaces being constructed as surfaces of rotation, characterised in that the joint surface (3) of the acetabular cavity (2) or the joint surface (4) of the joint head (1) is constructed as a Pascal's curve (worm) in meridian section.

2. An artificial hip joint according to claim 1, characterised in that the meridian section of the joint surface (4) of the joint head (1) is a Pascal's curve with a loop.

3. An artificial hip joint according to claim 1 or 2, characterised in that the meridian section of the joint surface (3) of the acetabular cavity (2) is an extended Pascal's curve.

4. An artificial hip joint according to one of claims 1 to 3, characterised in that the meridian section of the joint surface (3) of the acetabular cavity (2) and the meridian section of the joint surface (4) of the joint head (1) are Pascal's curves with interchanged parameters, i.e. if the meridian section of the hip head (1) complies with the equation r = a + b cos φ, then the meridian section of the hip socket (2) satisfies the equation r' = b + a cos φ, r and r' and φ being the polar co-ordinates of a point of the meridian section of the hip head or hip socket respectively while φ is the angle between the axis of symmetry of the Pascal's curve and the radius vector at a point on the curve, a and b being the given parameters.

5. An artificial hip joint according to claim 4, characterised in that the ratio of a:b < 1, the parameter a preferably being equal to 18.75 mm while the parameter b is 21.5 mm.

6. An artificial hip joint according to one of claims 1 to 5, characterised in that between the axis of the femur neck (11) and the axis of rotation (10) of the joint head (1) (viewed in plan), there is an angular deviation (a) which is preferably 10.7° +/-3_{°}.

## Revendications

1. Articulation de hanche artificielle pour remplacer la tête d'articulation naturelle et/ou la cavité d'articulation naturelle, les surfaces d'articulation artificielles étant réalisées sous la forme de surfaces de révolution, caractérisée en ce que la surface d'articulation (3) de la cavité d'articulation (2) ou, selon le cas, la surface d'articulation (4) de la tête d'articulation (1) est réalisée, en coupe méridienne, sous la forme d'une courbe de Pascal (hélice).

2. Articulation de hanche artificielle selon la revendication 1, caractérisée en ce que la section méridienne de la surface d'articulation (4) de la tête d'articulation (1) est une courbe de Pascal avec boucle.

3. Articulation de hanche artificielle selon la revendication 1 ou 2, caractérisée en ce que la section méridienne de la surface d'articulation (3) de la cavité d'articulation (2) est une courbe de Pascal déployée.

4. Articulation de hanche artificielle selon l'une des revendications 1 à 3, caractérisée en ce que la section méridienne de la surface d'articulation (3) de la cavité d'articulation (2) et la section méridienne de la surface d'articulation (4) de la tête d'articulation (1) sont des courbes de Pascal avec des paramètres mutuellement permutés, c'est-à-dire que si la section méridienne de la tête de hanche (1) suit la relation r = a + b cos(phi), la section méridienne de la cavité de hanche (2) suit la relation r' = b + a cos(phi), r ou r' et phi étant les coordonnées polaires d'un point de la section méridienne, respectivement de la tête d'articulation ou de la cavité d'articulation, phi étant l'angle entre l'axe de symétrie de la courbe de Pascal et le rayon vecteur en un point de la courbe, tandis qu'a et b sont les paramètres alloués.

5. Articulation de hanche artificielle selon la revendication 4, caractérisée en ce que le rapport a/b est inférieur à 1, le paramètre a étant de préférence égal à 18,75 mm et le paramètre b à 21,5 mm.

6. Articulation de hanche artificielle selon l'une des revendications 1 à 5, caractérisée en ce qu'un écart angulaire (alpha), qui est de préférence égal à 10,7 +/- 3° , est présent entre l'axe du col du fémur (11) et l'axe de rotation (10) de la tête d'articulation (1) (en vue de dessus).
